(19) Europäisches Patentamt
European Patent Office
Office européen des brevets

(11) **EP 3 669 763 B1**

(12) **EUROPEAN PATENT SPECIFICATION**

(45) Date of publication and mention
of the grant of the patent:
**15.11.2023  Bulletin 2023/46**

(21) Application number: **19216050.5**

(22) Date of filing: **13.12.2019**

(51) International Patent Classification (IPC):
**A61B 5/021** *(2006.01)*      **A61B 5/024** *(2006.01)*

(52) Cooperative Patent Classification (CPC):
**A61B 5/02108; A61B 5/02416; A61B 5/7242**

(54) **APPARATUS AND METHOD FOR ESTIMATING CARDIOVASCULAR INFORMATION**

VORRICHTUNG UND VERFAHREN ZUR SCHÄTZUNG VON KARDIOVASKULÄREN INFORMATIONEN

APPAREIL ET PROCÉDÉ D'ESTIMATION D'INFORMATIONS CARDIOVASCULAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2018  KR 20180167338**

(43) Date of publication of application:
**24.06.2020  Bulletin 2020/26**

(73) Proprietor: **Samsung Electronics Co., Ltd. Gyeonggi-do 16677 (KR)**

(72) Inventors:
• **KWON, Ui Kun**
**Hwaseong-si, Gyeonggi-do (KR)**

• **KIM, Youn Ho**
**Hwaseong-si, Gyeonggi-do (KR)**
• **PARK, Chang Soon**
**Chungju-si, Chungcheongbuk-do (KR)**

(74) Representative: **Grünecker Patent- und Rechtsanwälte
PartG mbB
Leopoldstraße 4
80802 München (DE)**

(56) References cited:
WO-A1-2018/172958      JP-A- 2000 237 153
JP-A- 2000 300 526      US-A1- 2006 074 322
US-A1- 2009 024 012      US-A1- 2009 326 393

## Description

## BACKGROUND

### 1. Field

Apparatuses and methods consistent with example embodiments relate to estimating cardiovascular information.

### 2. Description of the Related Art

Healthcare technology is receiving a lot of attention as society is rapidly aging, causing social problems such as increasing healthcare costs and the like. Accordingly, not only medical devices for use in hospitals or medical examination institutions, but also small medical devices that individuals can carry are being developed. Furthermore, such small medical devices are provided in the form of wearable devices worn by users to measure cardiovascular health states, thereby enabling users to directly measure and manage cardiovascular health states.

Therefore, much research has been conducted recently on methods to estimate blood pressure by analyzing bio-signals, so as to manufacture the devices in a compact size.

US 2009 326 393 A1 provides systems and methods for non-invasive continuous blood pressure determination. It is disclosed that a PPG signal is received and locations of pulses within the PPG signal are identified. It is further disclosed that an area within a particular pulse is measured, wherein the area is of just the upstroke, downstroke or the entire pulse. It is further disclosed that the area is measured relative to a time-domain axis or a baseline of the pulse, wherein the pulse is split into multiple sections and the area of each section is measured. It is disclosed that the area of one portion of the pulse correspond to systolic blood pressure while the area of another portion correspond to diastolic blood pressure. It is further disclosed that empirical data is used to determine blood pressure from the measured area by applying calibration data measured by a suitable device.

US 2006 074 322 A1 discloses a cuff-based method for the measurement of systolic blood pressure (SBP) by measuring photoplethysmographic (PPG) signals in peripheral blood vessels distal to the cuff and to a method for cuffless measurement of SBP by analyzing PPG signals in peripheral blood vessels, after suitable calibration by the cuff-based PPG method for the measurement of SBP

JP 2000 300 526 A dislcoses a sphygmomanometer including a means for performing a calculation using information and a means for determining a blood pressure using the calculation result by this means, and a means for extracting a pulse wave during measurement and a means for extracting the pulse wave by this means.

US 2009 024 012 A1 discloses a method and apparatus for measuring blood oxygen saturation by using spectrophotometry under a condition of low perfusion.

WO 2018 172 958 A1 discloses a system for detecting vital physiological parameters of a subject. The system comprises a support means which can be associated with the subject and are connected to acquisition means configured to acquire synchronously an ECG signal and a PPG signal of the subject. The system further comprises a processing means which is connected to the acquisition means and comprises a systolic and diastolic arterial pressure module configured to calculate a vital physiological parameter related to systolic and diastolic arterial pressure

JP 2000 237 153 A discloses a non-invasive continuous blood pressure estimation device for continuously estimating the intra-arterial blood pressure of a living body in a non-invasive manner, and continuously determining circulatory information obtained from the circulatory system of the living body in a non-invasive manner.

## SUMMARY

It is therefore the object of the present invention to provide a method and a corresponding apparatus for estimating cardiovascular information in a more accurate manner.

This object is solved by the subject matter of the independent claims.

Embodiments are defined by the dependent claims.

One or more example embodiments provide an apparatus and method for estimating cardiovascular information by stably extracting cardiovascular feature values even when the quality of a bio-signal is poor.

According to an aspect of an example embodiment, there is provided an apparatus for estimating cardiovascular information, including: a memory storing instructions; a processor configured to execute the instructions to: obtain a pulse wave signal of an object; determine an area under a pulse wave signal and above a reference point on the pulse wave signal; and estimate cardiovascular information of the object based on the determined area of the pulse wave signal.

The apparatus may further include a pulse wave signal obtainer including: a light source configured to emit light onto the object; and a photodetector configured to obtain the pulse wave signal by receiving the light returning from the object. wherein the pulse wave obtainer may be configured to provide the pulse wave signal to the processor.

The processor may be further configured to execute the instructions to determine a time of a maximum point of a propagation wave component included in the pulse wave signal, and determine a point of the pulse wave signal, which corresponds to the time of the maximum point of the propagation wave component, as the reference point.

The processor may be further configured to execute the instructions to determine a secondary differential signal of the pulse wave signal, determine a local minimum point in a predetermined interval of the secondary differential signal, and determine a point of the pulse wave signal, which corresponds to a time of the local minimum point, as the reference point.

The processor may be further configured to execute the instructions to detect a pulse wave start point or a maximum ascending slope point from the pulse wave signal, and determine, as the reference point, one of a point after a first duration from the pulse wave start point, the maximum ascending slope point, and a point before or after a second duration from the maximum ascending slope point.

The processor may be further configured to execute the instructions to detect, as the pulse wave start point, a minimum point of the pulse wave signal, or an intersection point between a tangent line at the maximum ascending slope point of the pulse wave signal and a height of the minimum point of the pulse wave signal.

The processor may be further configured to execute the instructions to normalize the pulse wave signal or the determined area of the pulse wave signal based on at least one of a pulse wave signal amplitude corresponding to a time of a maximum point of a propagation wave component included in the pulse wave signal, a pulse wave signal amplitude at a point after a third duration from a pulse wave start point, a pulse wave signal amplitude at a maximum ascending slope point, a pulse wave signal amplitude at a point before or after a fourth duration from the maximum ascending slope point, and a duration of the pulse wave signal at a point higher than the reference point.

The processor may be further configured to execute the instructions to remove an area of a propagation wave component, included in the determined area of the pulse wave signal, from the determined area of the pulse wave signal.

The cardiovascular information may include at least one of blood pressure, vascular age, arterial stiffness, vascular compliance, blood glucose, blood triglyceride, and total peripheral resistance.

The processor may be further configured to execute the instructions to estimate the cardiovascular information by using the determined area of the pulse wave signal as a cardiovascular feature value.

The processor may be further configured to execute the instructions to estimate the cardiovascular information by using a cardiovascular information estimation model which defines a relationship between the cardiovascular feature value and the cardiovascular information.

The processor may be further configured to execute the instructions to remove noise from the obtained pulse wave signal.

According to an aspect of another example embodiment, there is provided a method of estimating cardiovascular information, including: obtaining a pulse wave signal of an object; determining an area under a pulse wave signal and above a reference point on the pulse wave signal; and estimating cardiovascular information of the object based on the determined area of the pulse wave signal.

The obtaining the pulse wave signal of the object may include: emitting light onto the object; and obtaining the pulse wave signal by receiving the light returning from the object.

The determining the area of the pulse wave signal may include: determining a time of a maximum point of a propagation wave component included in the pulse wave signal; and determining a point of the pulse wave signal, which corresponds to the determined time of the maximum point of the propagation wave component, as the reference point.

The determining the time of the maximum point of the propagation wave component may include: determining a secondary differential signal of the pulse wave signal; determining a local minimum point in a predetermined interval of the secondary differential signal; and determining a time of the determined local minimum point to be the time of the maximum point of the propagation wave component.

The determining the area of the pulse wave signal may include: detecting a pulse wave start point or a maximum ascending slope point from the pulse wave signal; and determining, as the reference point, one of a point after a first duration from the pulse wave start point, the maximum ascending slope point, and a point before or after a second duration from the maximum ascending slope point.

The detecting the pulse wave start point or the maximum ascending slope point may include detecting, as the pulse wave start point, a minimum point of the pulse wave signal, or an intersection point between a tangent line at the maximum ascending slope point of the pulse wave signal and a height of the minimum point of the pulse wave signal.

The method may further include normalizing the pulse wave signal or the determined area of the pulse wave signal based on at least one of a pulse wave signal amplitude corresponding to a time of a maximum point of a propagation wave component included in the pulse wave signal, a pulse wave signal amplitude at a point after a third duration from a pulse wave start point, a pulse wave signal amplitude at a maximum ascending slope point, a pulse wave signal amplitude at a point before or after a fourth duration from the maximum ascending slope point, and a duration of the pulse wave signal at a point higher than the reference point.

The method may further include removing an area of a propagation wave component, included in the determined area of the pulse wave signal, from the determined area of the pulse wave signal.

The cardiovascular information may include at least one of blood pressure, vascular age, arterial stiffness, vascular compliance, blood glucose, blood triglyceride, and total peripheral resistance.

The estimating the cardiovascular information may include estimating the cardiovascular information by using the determined area of the pulse wave signal as a cardiovascular feature value.

The estimating the cardiovascular information may include estimating the cardiovascular information by using a cardiovascular information estimation model which defines a relationship between the cardiovascular feature value and the cardiovascular information.

The method may include removing noise from the obtained pulse wave signal.

According to an aspect of another example embodiment, there is provided an apparatus for estimating cardiovascular information, the apparatus including: a memory storing instructions; and a processor configured to execute the instructions to: obtain a pulse wave signal of an object; determine a secondary differential signal of the pulse wave signal; in response to detecting a local minimum point in a predetermined interval of the secondary differential signal, estimate cardiovascular information in a first operation mode; and in response to no local minimum point being detected in the predetermined interval of the secondary differential signal, estimate the cardiovascular information in a second operation mode different from the first operation mode.

The apparatus may further include a pulse wave signal obtainer including: a light source configured to emit light onto the object; and a photodetector configured to obtain the pulse wave signal by receiving the light returning from the object, wherein the pulse wave obtainer may be configured to provide the pulse wave signal to the processor.

In the first operation mode, the processor may be further configured to execute the instructions to extract at least one feature value by analyzing the secondary differential signal, and estimate the cardiovascular information by using the extracted at least one feature value as a cardiovascular feature value.

In the first operation mode, the processor may be further configured to execute the instructions to: determine a time of a maximum point of a propagation wave component and a time of a maximum point of a reflection wave component by analyzing the secondary differential signal; determine a pulse wave signal amplitude corresponding to each time of the maximum point of the propagation wave component and the maximum point of the reflection wave component; and extract the at least one feature value by combining at least one of the time of the maximum point of the propagation wave component, a pulse wave signal amplitude corresponding to the time of the maximum point of the propagation wave component, the time of the maximum point of the reflection wave component, and a pulse wave signal amplitude corresponding to the time of the maximum point of the reflection wave component.

In the second operation mode, the processor may be further configured to execute the instructions to determine an area under the pulse wave signal and above a reference point on the pulse wave signal, and estimate the cardiovascular information of the object by using the determined area of the pulse wave signal as a cardiovascular feature value.

The processor may be further configured to execute the instructions to detect a pulse wave start point or a maximum ascending slope point from the pulse wave signal, and determine, as the reference point, one of a point after a first duration from the pulse wave start point, the maximum ascending slope point, and a point before or after a second duration from the maximum ascending slope point.

The processor may be further configured to execute the instructions to detect, as the pulse wave start point, a minimum point of the pulse wave signal, or an intersection point between a tangent line at the maximum ascending slope point of the pulse wave signal and a height of the minimum point of the pulse wave signal.

In the second operation mode, the processor may be further configured to execute the instructions to normalize the pulse wave signal or the determined area of the pulse wave signal based on at least one of a pulse wave signal amplitude at the point after a first duration from a pulse wave start point, a pulse wave signal amplitude at a maximum ascending slope point, a pulse wave signal amplitude at a point before or after a second duration from the maximum ascending slope point, and a duration of the pulse wave signal at a point higher than the reference point.

According to an aspect of another example embodiment, there is provided a method of estimating cardiovascular information, including: obtaining a pulse wave signal of an object; determining a secondary differential signal of the pulse wave signal; in response to detecting a local minimum point in a predetermined interval of the secondary differential signal, estimating cardiovascular information in a first operation mode; and in response to no local minimum point being detected in the predetermined interval of the secondary differential signal, estimating the cardiovascular information in a second operation mode different from the first operation mode.

The obtaining the pulse wave signal of the object may include: emitting light onto the object; and obtaining the pulse wave signal by receiving the light returning from the object.

The estimating the cardiovascular information in the first operation mode may include: extracting at least one feature value by analyzing the secondary differential signal; and estimating the cardiovascular information by using the extracted at least one feature value as a cardiovascular feature value.

The extracting the at least one feature value may include: determining a time of a maximum point of a propagation wave component and a time of a maximum point of a reflection wave component by analyzing the secondary differential signal; determining a pulse wave signal amplitude corresponding to each time of the maximum point of the propagation wave component and the maximum point of the reflection wave component; and extracting the at least one feature value by combining at least one of the time of the maximum point of the propagation wave component, a pulse wave signal amplitude corresponding to the time of the maximum point of the propagation wave component, the time of the maximum point of the reflection wave component, and a pulse wave signal amplitude corresponding to the time of the maximum point of the reflection wave component.

The estimating the cardiovascular information in the second operation mode may include: determining an area under the pulse wave signal and above a reference point on the pulse wave signal; and estimating the cardiovascular information of the object by using the determined area of the pulse wave signal as a cardiovascular feature value.

The determining the area of the pulse wave signal may include: detecting a pulse wave start point or a maximum ascending slope point from the pulse wave signal; and determining, as the reference point, one of a point after a first duration from the pulse wave start point, the maximum ascending slope point, and a point before or after a second duration from the maximum ascending slope point.

The detecting the pulse wave start point or the maximum ascending slope point may include detecting, as the pulse wave start point, a minimum point of the pulse wave signal, or an intersection point between a tangent line at the maximum ascending slope point of the pulse wave signal and a height of the minimum point of the pulse wave signal.

The estimating the cardiovascular information in the second operation mode may include normalizing the pulse wave signal or the determined area of the pulse wave signal based on at least one of a pulse wave signal amplitude at the point after a first duration from a pulse wave start point, a pulse wave signal amplitude at a maximum ascending slope point, a pulse wave signal amplitude at a point before or after a second duration from the maximum ascending slope point, and a duration of the pulse wave signal at a point higher than the reference point.

BRIEF DESCRIPTION OF THE DRAWINGS

The above and/or other aspects will be more apparent by describing certain example embodiments, with reference to the accompanying drawings, in which:

FIG. 1 is a diagram illustrating an example of a pulse wave signal;
FIG. 2 is a diagram illustrating an apparatus for estimating cardiovascular information according to an example embodiment;
FIG. 3 is a diagram illustrating a pulse wave signal obtainer according to an example embodiment;
FIG. 4 is a diagram explaining an example of determining a reference point;
FIG. 5 is a diagram explaining another example of determining a reference point;
FIG. 6 is a diagram explaining an example of a cardiovascular feature value;
FIG. 7 is a diagram illustrating an apparatus for estimating cardiovascular information according to another example embodiment;
FIG. 8 is a diagram illustrating a method of estimating cardiovascular information according to an example embodiment;
FIG. 9 is a diagram illustrating method of determining an area of a pulse wave signal which is at a point higher than a reference point according to an example embodiment;
FIG. 10 is a diagram illustrating a method of determining an area of a pulse wave signal which is at a point higher than a reference point according to another example embodiment;
FIG. 11 is a diagram illustrating a method of estimating cardiovascular information according to another example embodiment;
FIG. 12 is a diagram illustrating an apparatus for estimating cardiovascular information according to another example embodiment;
FIG. 13 is a diagram explaining a first feature and a second feature extracted in a first operation mode;
FIG. 14 is a diagram explaining a method of obtaining $P_n$ ($P_1$, $P_2$, $P_3$) and $T_n$ ($T_1$, $T_2$, $T_3$) of FIG. 13;
FIG. 15 is a diagram explaining a method of obtaining $P_{max}$ and $T_{max}$ of FIG. 13;
FIG. 16 is a diagram illustrating a method of estimating cardiovascular information according to another example embodiment;
FIG. 17 is a diagram illustrating a method of estimating cardiovascular information in a first operation mode according to an example embodiment; and
FIG. 18 is a diagram illustrating a method of estimating cardiovascular information in a second operation mode according to an example embodiment.

DETAILED DESCRIPTION

Example embodiments are described in greater detail below with reference to the accompanying drawings. Throughout the drawings and the detailed description, unless otherwise described, the same drawing reference numerals will be understood to refer to the same elements, features, and structures. The relative size and depiction of these elements may be exaggerated for clarity, illustration, and convenience.

In the following description, like drawing reference numerals are used for like elements, even in different drawings. The matters defined in the description, such as detailed construction and elements, are provided to assist in a comprehensive understanding of the example embodiments. However, it is apparent that the example embodiments can be practiced without those specifically defined matters. Also, well-known functions or constructions are not described in detail since they would obscure the description with unnecessary detail.

Process steps described herein may be performed differently from a specified order, unless a specified order is clearly stated in the context of the disclosure. That is, each step may be performed in a specified order, at substantially the same time, or in a reverse order.

It will be understood that, although the terms first, second, etc. may be used herein to describe various elements, these elements should not be limited by these terms. These terms are only used to distinguish one element from another. Any references to singular may include plural unless expressly stated otherwise. In the present specification, it should be understood that the terms, such as 'including' or 'having,' etc., are intended to indicate the existence of the features, numbers, steps, actions, components, parts, or combinations thereof disclosed in the specification, and are not intended to preclude the possibility that one or more other features, numbers, steps, actions, components, parts, or combinations thereof may exist or may be added.

Expressions such as "at least one of," when preceding a list of elements, modify the entire list of elements and do not modify the individual elements of the list. For example, the expression, "at least one of a, b, and c," should be understood as including only a, only b, only c, both a and b, both a and c, both b and c, all of a, b, and c, or any variations of the aforementioned examples.

Further, components that will be described in the present disclosure are discriminated merely according to functions mainly performed by the components. That is, two or more components which will be described later can be integrated into a single component. Furthermore, a single component which will be explained later can be separated into two or more components. Moreover, each component which will be described can additionally perform some or all of a function executed by another component in addition to the main function thereof. Some or all of the main function of each component can be carried out by another component. Each component may be implemented as hardware, software, or a combination of both.

An apparatus for estimating cardiovascular information which will be described in the present disclosure may be implemented as a software module or may be manufactured in the form of a hardware chip to be embedded in an electronic apparatus. In this case, examples of the electronic apparatus may include a cellular phone, a smartphone, a tablet PC, a laptop computer, a personal digital assistant (PDA), a portable multimedia player (PMP), a navigation, an MP3 player, a digital camera, a wearable device, and the like; and examples of a wearable device may include a wristwatch-type wearable device, a wristband-type wearable device, a ring-type wearable device, a waist belt-type wearable device, a necklace-type wearable device, an ankle band-type wearable device, a thigh band-type wearable device, a forearm band-type wearable device, and the like. However, the electronic device is not limited to the above examples, and the wearable device is neither limited thereto.

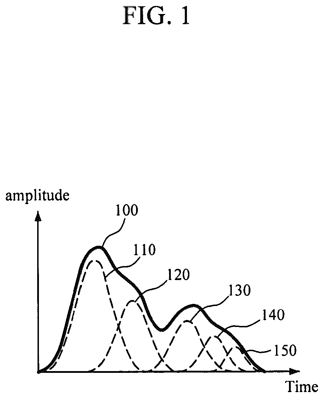

FIG. 1 is a diagram illustrating an example of a pulse wave signal. More specifically, FIG. 1 illustrates an example of a photoplethysmogram (PPG) signal.

Referring to FIG. 1, a waveform of the PPG signal 100 may be obtained from a subject, and may be formed by a superposition of a propagation wave component 110, and reflection wave components 120 to 150. The propagation wave component 110 is derived from blood departing from the heart of the subject and moving toward the distal end portions of the body of the subject, and the reflection wave components 120 to 150 are derived from blood returning back from the distal end portions of the subject. In the illustrated example, the PPG signal 100 is composed of a superposition of five component pulses 110 to 150.

Blood pressure may be determined by cardiac output (CO), which is the total volume of blood ejected by the heart per unit time, and total peripheral resistance (TPR), and may be represented by the following Equation 1.

[Equation 1]

$$BP = CO \times TPR$$

Here, BP denotes a blood pressure difference between the left ventricle and the right atrium, CO denotes the cardiac output, and TPR denotes the total peripheral resistance.

That is, in the case where the CO changes and/or the TPR changes, blood pressure also changes. Accordingly, by extracting feature values associated with the CO and/or feature values associated with the TPR from the PPG signal 100, blood pressure may be estimated by using the extracted feature values.

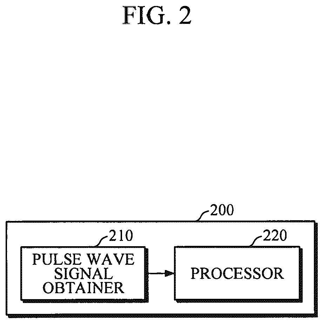

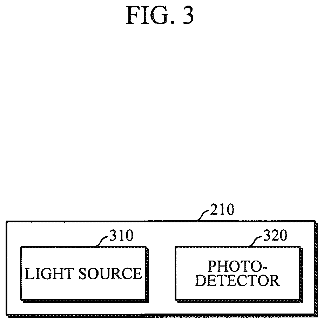

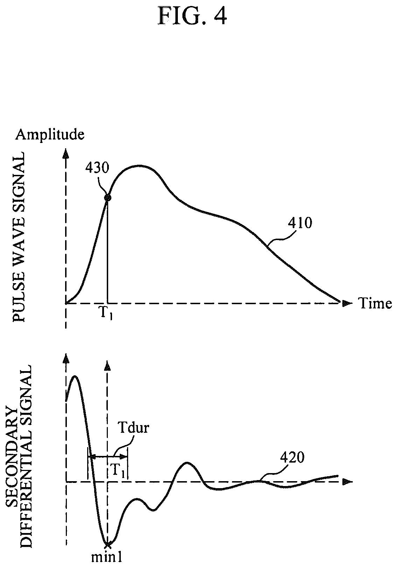

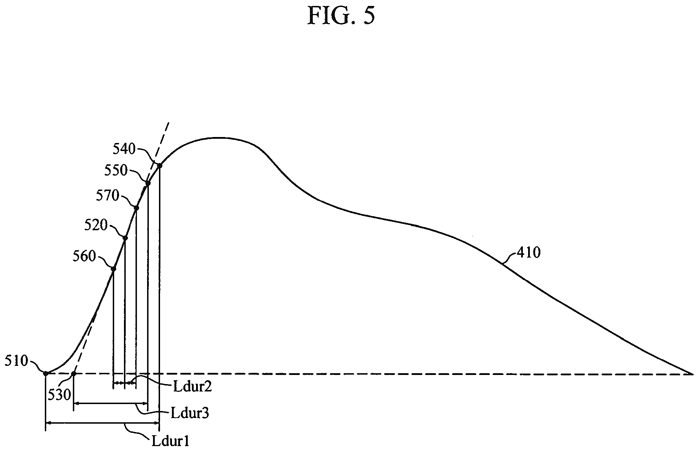

FIG. 2 is a diagram illustrating an apparatus for estimating cardiovascular information according to an example embodiment; FIG. 3 is a diagram illustrating a pulse wave signal obtainer according to an example embodiment; FIG. 4 is a diagram explaining an example of determining a reference point; and FIG. 5 is a diagram explaining another example of determining a reference point.

Referring to FIG. 2, a cardiovascular information estimating apparatus 200 includes a pulse wave signal obtainer 210 and a processor 220. Here, the processor 220 may include one or more processors, a memory, or a combination thereof.

The pulse wave signal obtainer 210 may obtain a pulse wave signal of an object. Here, the pulse wave signal may be a PPG signal.

In one embodiment, the pulse wave signal obtainer 210 may receive the pulse wave signal of the object from an external device which measures and/or stores pulse wave signals. In this case, the pulse wave signal obtainer 210 may communicate with the external device by using various communication techniques such as Bluetooth communication, Bluetooth Low Energy (BLE) communication, Near Field Communication (NFC), WLAN communication, Zigbee communication, Infrared Data Association (IrDA) communication, Wi-Fi Direct (WFD) communication, Ultra Wideband (UWB) communication, Ant+ communication, WIFI communication, Radio Frequency Identification (RFID) communication, and the like. However, this is merely exemplary and the communication is not limited thereto.

In another example, the pulse wave signal obtainer 210 may emit light of a predetermined wavelength onto the object, and may measure the pulse wave signal by receiving light reflected or scattered from the object. The pulse wave signal obtainer 210 may be implemented by a PPG sensor. As illustrated in FIG. 2, the pulse wave signal obtainer 210 may include a light source 310 and a photodetector 320.

The light source 310 may emit visible rays or near-infrared rays onto the object. However, wavelengths of light to be emitted by the light source 310 may vary according to a purpose of measurement. Further, the light source 310 is not necessarily a single light-emitting body, and may be formed as an array of a plurality of light-emitting bodies. The light source 310 may include a light emitting diode (LED), a laser diode, a fluorescent body, and the like.

The photodetector 320 may measure a pulse wave signal by receiving light reflected or scattered from the object. The photodetector 320 may include a photo diode, a photo transistor (PTr), a charge-coupled device (CCD), and the like. The photodetector 320 is not necessarily a single device, and may be formed as an array of a plurality of devices.

There may be various numbers and arrangements of the light source and the photodetector, and the number and arrangement thereof may vary according to a purpose of measurement and the size and shape of the electronic device in which the pulse wave signal obtainer 210 is mounted, and the like.

The processor 220 may control the overall operation of the cardiovascular information estimation apparatus 200.

At predetermined intervals or in response to a user's request, the processor 220 may control the pulse wave signal obtainer 210 to receive the pulse wave signal of the object.

The processor 220 may remove noise from the obtained pulse wave signal. For example, the processor 220 may remove noise from the pulse wave signal by using various filtering techniques such as a band pass filter, a moving average, and the like.

The processor 220 may determine a reference point on the pulse wave signal. The reference point may be a point of reference for determining an area to be used as a cardiovascular feature value.

In one embodiment, the processor 220 may determine a time of a maximum point of the propagation wave component 110 included in the pulse wave signal 410, and may determine a point of the pulse wave signal, 410 which corresponds to the time of the maximum point of the propagation wave component 110, as the reference point. For example, referring to FIG. 4, the processor 220 may determine a secondary differential signal 420 of a pulse wave signal 410, and may determine a local minimum point mini in a predetermined interval Tdur of the secondary differential signal 420. Further, the processor 220 may determine a time T1 of the determined local minimum point mini to be the time of the maximum point of the propagation wave component, and may determine a point 430 of the pulse wave signal 410 at the time T1, as the reference point. In this case, the predetermined interval Tdur may be an interval in which the propagation wave component 110 included in the pulse wave signal 410 may appear, and may be obtained experimentally as a preset value.

In another embodiment, the processor 220 may detect a pulse wave start point or a maximum ascending slope point from the pulse wave signal. The processor 220 may determine, as the reference point, one of the following: a point after a first duration (e.g., predetermined duration Ldur1 illustrated in FIG. 5) from the pulse wave start point, the maximum ascending slope point, and a point before/after a second duration (e.g., a predetermined duration Ldur2 illustrated in FIG. 5) from the maximum ascending slope point. Here, the first duration and the second duration may be predetermined according to specifications of the cardiovascular information estimating apparatus 200.

For example, referring to FIG. 5, the processor 220 may detect pulse wave start points 510 and 530 or the maximum ascending slope point 520 from the pulse wave signal 410. The pulse wave start point 510 may be a minimum point of the pulse wave signal 410. The pulse wave start point 530 may indicate an intersection point between a tangent line at the maximum ascending slope point 520 of the pulse wave signal 410 and a height of the minimum point 510 of the pulse wave signal 410. The processor 220 may detect, as the pulse wave start point, the minimum point 510 of the pulse wave signal 410, or the intersection point 530 between the tangent line at the maximum ascending slope point 520 of the pulse wave signal 410 and the height of the minimum point 510 of the pulse wave signal 410.

The processor 220 may determine, as the reference point, one of the following: a point 540 after a predetermined duration Ldur1 from the pulse wave start point 510; a point 550 after a predetermined duration Ldur2 from the pulse wave start point 530, the maximum ascending slope point 520, and points 560 and 570 before/after a predetermined duration Ldur3 from the maximum ascending slope point 520.

The processor 220 may normalize the pulse wave signal 410. In one embodiment, the processor 220 may normalize the pulse wave signal 410 based on at least one of the following: a pulse wave signal amplitude corresponding to the time of the maximum point of the propagation wave component 110 included in the pulse wave signal 410; a pulse wave signal amplitude at a point after a third duration from the pulse wave start point; a pulse wave signal amplitude at the maximum ascending slope point 520; a pulse wave signal amplitude at a point before/after a fourth duration from the maximum ascending slope point 520; and a duration of the pulse wave signal 410 at a point higher than the reference point. Here, the third duration and the fourth duration may be predetermined according to specifications of the cardiovascular information estimating apparatus 200, and may be the same as or different from the aforementioned first duration and second duration, respectively.

The processor 220 may determine an area of the pulse wave signal 410 which is at the point higher than the reference point on the normalized pulse wave signal, and may estimate cardiovascular information by using the determined area as a cardiovascular feature value. In this case, the cardiovascular information may include blood pressure, vascular age, arterial stiffness, vascular compliance, blood glucose, blood triglyceride, total peripheral resistance, and the like. In one embodiment, the processor 220 may use a cardiovascular information estimation model which defines a relationship between a cardiovascular feature value and cardiovascular information.

In one embodiment, in order to consider only reflection wave component information, the processor 220 may remove an area of the propagation wave component 110, included in the area of the pulse wave signal 410 at the point higher than the reference point, from the area of the pulse wave signal 410, and may estimate cardiovascular information by using the area, from which an effect of the propagation wave component is removed, as a cardiovascular feature value. For example, the processor 220 may determine a time of the maximum point of the propagation wave component included in the pulse wave signal 410, and may determine a point of the pulse wave signal 410 which corresponds to the time. Further, the processor 220 may determine an area of the propagation wave component 110 based on the determined point of the pulse wave signal 410 and the reference point; and may remove the area of the propagation wave component 110 from the area of the pulse wave signal 410 which is at the point higher than the reference point. The processor 220 may improve the accuracy of a blood pressure estimation since the blood pressure estimation is performed based on the area under the curve of the pulse wave signal 410 which is robust against changes in the maximum amplitude of the pulse wave signal 410 and a width of the pulse wave signal above a reference amplitude line (or the reference point).

In addition, upon normalizing the pulse wave signal 410 as described above, the processor 220 may also calculate an area of the pulse wave signal 410 which is at the point higher than a reference point on the normalized pulse wave signal; and upon calculating the area of the pulse wave signal 410 which is at the point higher than the reference point, the processor 220 may also normalize the calculated area.

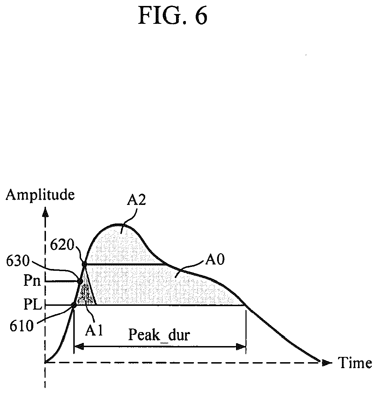

FIG. 6 is a diagram explaining an example of a cardiovascular feature value. In FIG. 6, a reference numeral 610 denotes a reference point; a reference numeral 620 denotes a point corresponding to a maximum point of a propagation wave component 110; and a reference numeral 630 denotes a normalization point for normalizing the pulse wave signal 410.

Referring to FIG. 6, the cardiovascular feature value according to an embodiment of the present disclosure may include values such as $A0/Pn$, $A0/(Pn*Peak\_dur)$, $(A0-A1)/Pn$, $(A0-A1)/(Pn*Peak\_dur)$, and the like. Here, $A0$ denotes an area under the pulse wave signal 410 which is at a point higher than the reference point 610; $Pn$ denotes an amplitude of the normalization point 630 used for normalization; $Peak\_dur$ denotes a duration of the pulse wave signal 410 at a point higher than the reference point 610 and a duration of the area determined as $A0$; and $A1$ denotes an area of the propagation wave component 110 included in the $A0$. $A2$ denotes an area under the pulse wave signal 410 and above the maximum point 620 of the propagation wave component 110. In this case, the points 610, 620, and 630 may be the same as or different from each other.

The processor 220 may perform a blood pressure estimation based on at least one of $A0/Pn$, $A0/(Pn*Peak\_dur)$, $(A0-A1)/Pn$, $(A0-A1)/(Pn*Peak\_dur)$, and $A2$.

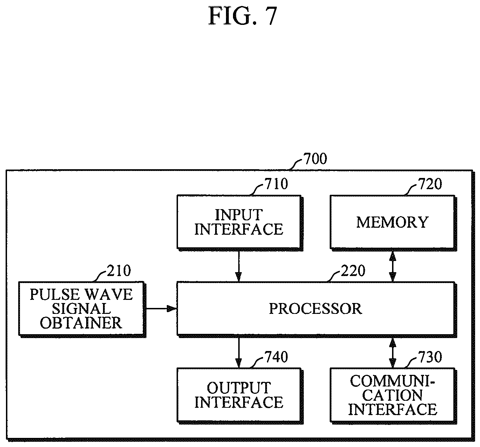

FIG. 7 is a diagram illustrating an apparatus for estimating cardiovascular information according to another example embodiment.

Referring to FIG. 7, the cardiovascular information estimating apparatus 700 includes the pulse wave signal obtainer 210, the processor 220, an input interface 710, a memory 720, a communication interface 730, and an output interface 740. Here, the pulse wave signal obtainer 210 and the processor 220 are described above with reference to FIGS. 2 to 6, such that detailed description thereof will be omitted.

The input interface 710 may receive input of various operation signals from a user. In the embodiment, the input interface 710 may include a keypad, a dome switch, a touch pad (static pressure/capacitance), a jog wheel, a jog switch, a hardware (H/W) button, and the like. Particularly, the touch pad, which forms a layer structure with a display, may be called a touch screen.

The memory 720 may store programs or commands for operation of the cardiovascular information estimating apparatus 700, and may store data input to and output from the cardiovascular information estimating apparatus 700. Further, the memory 720 may store the obtained pulse wave signal, the cardiovascular information estimation model, and the like. The memory 720 may include at least one storage medium of a flash memory type memory, a hard disk type memory, a multimedia card micro type memory, a card type memory (e.g., an SD memory, an XD memory, etc.), a Random Access Memory (RAM), a Static Random Access Memory (SRAM), a Read Only Memory (ROM), an Electrically Erasable Programmable Read Only Memory (EEPROM), a Programmable Read Only Memory (PROM), a magnetic memory, a magnetic disk, and an optical disk, and the like. Further, the cardiovascular information estimating apparatus 700 may operate an external storage medium, such as web storage and the like, which performs a storage function of the memory 720 on the Internet.

The communication interface730 may perform communication with an external device. For example, the communication interface730 may transmit, to the external device, the data input to the cardiovascular information estimating apparatus 700, data stored in and processed by the cardiovascular information estimating apparatus 700, and the like, or may receive, from the external device, various data useful for estimating cardiovascular information.

In this case, the external device may be medical equipment using the data input to the cardiovascular information estimating apparatus 700, the data stored in and processed by the cardiovascular information estimating apparatus 700, and the like, a printer to print out results, or a display to display the results. In addition, the external device may be a digital TV, a desktop computer, a cellular phone, a smartphone, a tablet PC, a laptop computer, a personal digital assistant (PDA), a portable multimedia player (PMP), a navigation, an MP3 player, a digital camera, a wearable device, and the like, but is not limited thereto.

The communication interface730 may communicate with an external device by using Bluetooth communication, Bluetooth Low Energy (BLE) communication, Near Field Communication (NFC), WLAN communication, Zigbee communication, Infrared Data Association (IrDA) communication, Wi-Fi Direct (WFD) communication, Ultra-Wideband (UWB) communication, Ant+ communication, WIFI communication, Radio Frequency Identification (RFID) communication, 3G communication, 4G communication, 5G communication, and the like. However, this is merely exemplary and is not intended to be limiting.

The output interface 740 may output the data input to the cardiovascular information estimating apparatus 700, the data stored in and processed by the cardiovascular information estimating apparatus 700, and the like. In one embodiment, the output interface 740 may output the data input to the cardiovascular information estimating apparatus 700, the data stored in and processed by the cardiovascular information estimating apparatus 700, and the like, by using at least one of an acoustic method, a visual method, and a tactile method. To this end, the output interface 740 may include a display, a speaker, a vibrator, and the like.

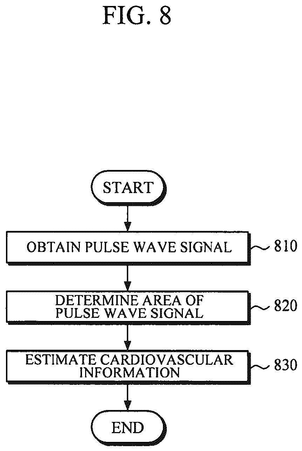

FIG. 8 is a diagram illustrating a method of estimating cardiovascular information according to an example embodiment according to an example embodiment. The cardiovascular information estimating method of FIG. 8 may be performed by the cardiovascular information estimating apparatus 200 or 700 of FIG. 2 or 7.

Referring to FIG. 8, the cardiovascular information estimating apparatus 200 or 700 may obtain a pulse wave signal 410 of an object in operation 810. Here, the pulse wave signal 410 may be a PPG signal. For example, the cardiovascular information estimating apparatus 200 or 700 may obtain the pulse wave signal 410 of the object by receiving the pulse wave signal 410 from an external device which measures and/or stores pulse wave signals, or may obtain the pulse wave signal 410 by emitting light of a predetermined wavelength and receiving light reflected or scattered from the object.

The cardiovascular information estimating apparatus 200 or 700 may determine an area of the pulse wave signal 410 which is at a point higher than a reference point on the pulse wave signal in operation 820. As described above, the reference point may be a point of reference for determining an area to be used as a cardiovascular feature value.

The cardiovascular information estimating apparatus 200 or 700 may estimate cardiovascular information of the object based on the determined area of the pulse wave signal 410 in operation 830. For example, the cardiovascular information estimating apparatus 200 or 700 may use the determined area of the pulse wave signal 410 as the cardiovascular feature value, and may estimate cardiovascular information of the object by using a cardiovascular information estimation model which defines a relationship between the cardiovascular feature value and cardiovascular information. The cardiovascular information may include blood pressure, vascular age, arterial stiffness, vascular compliance, blood glucose, blood triglyceride, total peripheral resistance, and the like.

In addition, in order to consider only reflection wave component information, the cardiovascular information estimating apparatus 200 or 700 may remove an area of the propagation wave component 110, included in the area of the pulse wave signal 410 at the point higher than the reference point, from the area of the pulse wave signal 410, and may estimate cardiovascular information by using the area, from which an effect of the propagation wave component 110 is removed, as a cardiovascular feature value. For example, the cardiovascular information estimating apparatus 200 or 700 may determine a time of a maximum point of the propagation wave component included in the pulse wave signal 410, and may determine a point of the pulse wave signal 410 which corresponds to the time. Further, the cardiovascular information estimating apparatus 200 or 700 may determine an area of the propagation wave component 110 based on the determined point of the pulse wave signal 410 and the reference point; and may remove the area of the propagation wave component 110 from the area of the pulse wave signal which is at the point higher than the reference point.

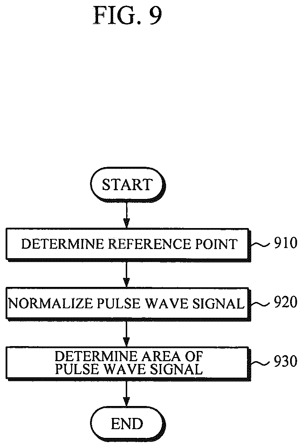

FIG. 9 is a diagram illustrating a method of determining an area of a pulse wave signal which is at a point higher than a reference point according to an example embodiment. The method of FIG. 9 may be an example of the determining of an area of a pulse wave signal 410 which is at a point higher than a reference point in operation 820 of FIG. 8.

Referring to FIG. 9, the cardiovascular information estimating apparatus 200 or 700 may determine a reference point on a pulse wave signal in operation 910.

In one embodiment, the cardiovascular information estimating apparatus 200 or 700 may determine a time of a maximum point of a propagation wave component 110 included in the pulse wave signal 410, and may determine a point of the pulse wave signal 410, which corresponds to the time, as the reference point. For example, the cardiovascular information estimating apparatus 200 or 700 may determine a secondary differential signal of the pulse wave signal 410, and may determine a local minimum point in a predetermined interval of the secondary differential signal. Further, the cardiovascular information estimating apparatus 200 or 700 may determine a time of the determined local minimum point to be the time of the maximum point of the propagation wave component 110, and may determine a point of the pulse wave signal, which corresponds to the time, as the reference point.

In another embodiment, the cardiovascular information estimating apparatus 200 or 700 may detect a pulse wave start point or a maximum ascending slope point from the pulse wave signal 410; and may determine, as the reference point, one of the following: a point after a first duration from the pulse wave start point, the maximum ascending slope point, and a point before/after a second duration from the maximum ascending slope point. In this case, the cardiovascular information estimating apparatus 200 or 700 may detect, as the pulse wave start point, a minimum point of the pulse wave signal 410, or an intersection point between a tangent line at the maximum ascending slope point of the pulse wave signal 410 and a height of the minimum point of the pulse wave signal 410.

The cardiovascular information estimating apparatus 200 or 700 may normalize the pulse wave signal 410 in operation 920. In one embodiment, the cardiovascular information estimating apparatus 200 or 700 may normalize the pulse wave signal 410 based on at least one of the following: a pulse wave signal amplitude corresponding to the time of the maximum point of the propagation wave component 110 included in the pulse wave signal 410; a pulse wave signal amplitude at a point after a third duration from the pulse wave start point; a pulse wave signal amplitude at the maximum ascending slope point; a pulse wave signal amplitude at a point before/after a fourth duration from the maximum ascending slope point; and a duration of the pulse wave signal 410 at a point higher than the reference point.

The cardiovascular information estimating apparatus 200 or 700 may determine an area of the pulse wave signal which is at the point higher than the reference point on the normalized pulse wave signal in operation 930.

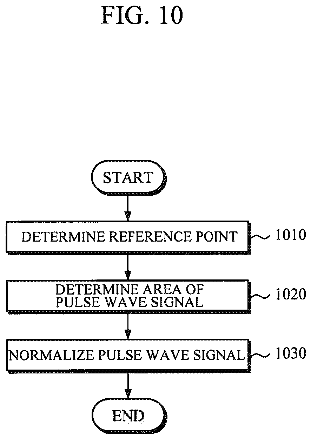

FIG. 10 is a diagram illustrating a method of determining an area of a pulse wave signal which is at a point higher than a reference point according to another example embodiment. The method of FIG. 10 may be another example of the determining of an area of a pulse wave signal 410 which is at a point higher than a reference point in operation 820 of FIG. 8.

Referring to FIG. 10, the cardiovascular information estimating apparatus 200 or 700 may determine a reference point on a pulse wave signal 410 in operation 1010.

The cardiovascular information estimating apparatus 200 or 700 may determine an area under the pulse wave signal 410 which is at a point higher than the reference point on the pulse wave signal 410 in operation 1020.

The cardiovascular information estimating apparatus 200 or 700 may normalize the pulse wave signal 410 in operation 1030. In one embodiment, the cardiovascular information estimating apparatus 200 or 700 may normalize the pulse wave signal 410 based on at least one of the following: a pulse wave signal amplitude corresponding to the time of the maximum point of the propagation wave component 110 included in the pulse wave signal 410; a pulse wave signal amplitude at a point after a third duration from the pulse wave start point; a pulse wave signal amplitude at the maximum ascending slope point; a pulse wave signal amplitude at a point before/after a fourth duration from the maximum ascending slope point; and a duration of the pulse wave signal at a point higher than the reference point.

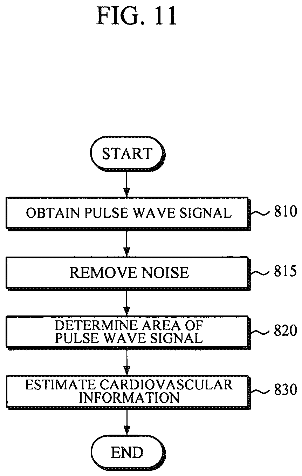

FIG. 11 is a diagram illustrating a method of estimating cardiovascular information according to another example embodiment. The cardiovascular information estimating method of FIG. 11 may be performed by the cardiovascular information estimating apparatus 200 or 700 of FIG. 2 or 7. The operations 810, 820, and 830 of FIG. 11 are described above with reference to FIG. 8, such that detailed description thereof will be omitted.

The cardiovascular information estimating apparatus 200 or 700 may remove noise from the obtained pulse wave signal in operation 815. For example, the cardiovascular information estimating apparatus 200 or 700 may remove noise from the pulse wave signal 410 by using various filtering techniques such as a band pass filter, a moving average, and the like.

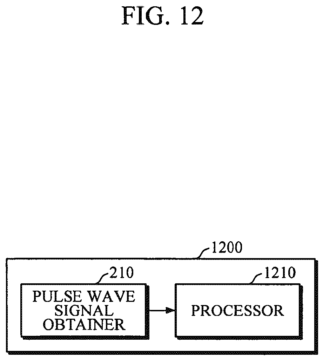

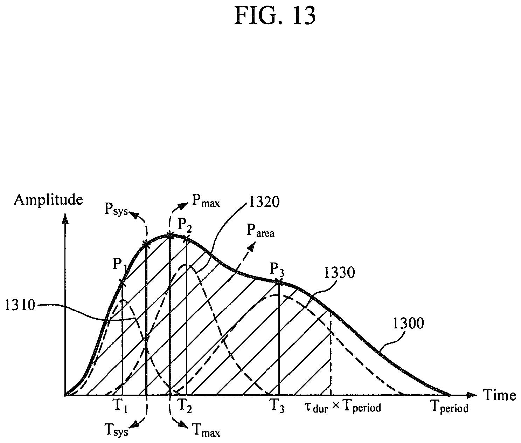

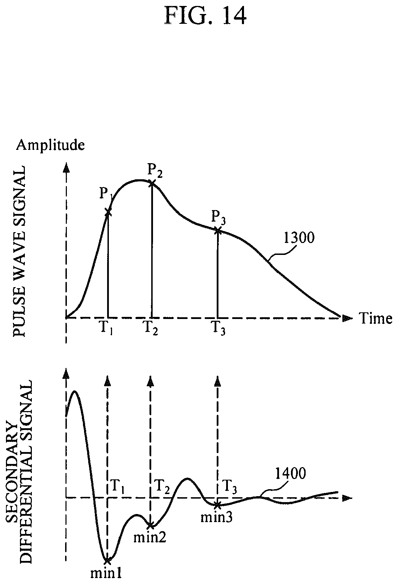

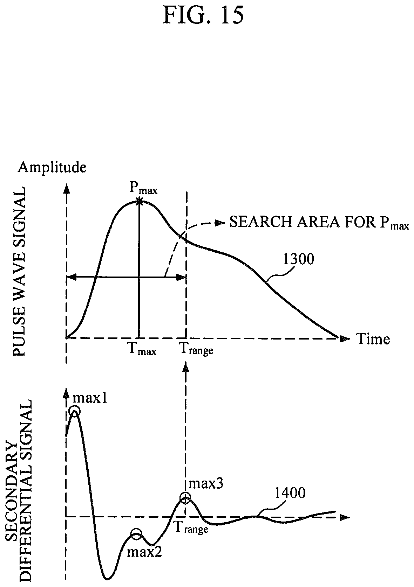

FIG. 12 is a diagram illustrating an apparatus for estimating cardiovascular information according to another example embodiment; FIG. 13 is a diagram explaining a first feature and a second feature extracted in a first operation mode; FIG. 14 is a diagram explaining a method of obtaining $P_n$ ($P_1$, $P_2$, $P_3$) and $T_n$ ($T_1$, $T_2$, $T_3$) of FIG. 13; and FIG. 15 is a diagram explaining a method of obtaining $P_{max}$ and $T_{max}$ of FIG. 13.

Referring to FIG. 12, a cardiovascular information estimating apparatus 1200 includes the pulse wave signal obtainer 210 and a processor 1210. Here, the processor 1210 may include one or more processors, a memory, or a combination thereof. The pulse wave signal obtainer 210 is described above with reference to FIGS. 2 and 3, such that detailed description thereof will be omitted.

The processor 1210 may control the overall operation of the cardiovascular information estimation apparatus 1200.

At predetermined intervals or in response to a user's request, the processor 1210 may control the pulse wave signal obtainer 210 to receive a pulse wave signal 1300 of an object.

The processor 1210 may remove noise from the obtained pulse wave signal 1300. For example, the processor 1210 may remove noise from the pulse wave signal 1300 by using various filtering techniques such as a band pass filter, a moving average, and the like.

The processor 1210 may determine a secondary differential signal 1400 of the pulse wave signal 1300; and if there is a local minimum point in a predetermined interval of the second differential signal 1400, the processor 1210 may estimate cardiovascular information in a first operation mode, and if there is no local minimum point in the predetermined interval of the second differential signal 1400, the processor 1210 may estimate cardiovascular information in a second operation mode. In this case, the predetermined interval may be an interval in which the propagation wave component included in the pulse wave signal 1300 may appear, and may be obtained experimentally as a preset value.

Hereinafter, the first operation mode and the second operation mode will be explained separately.

<First operation mode>

In the first operation mode, the processor 1210 may extract a first feature value and/or a second feature value from the pulse wave signal 1300. In this case, the first feature value may be associated with cardiac output, and the second feature value may be associated with total peripheral resistance. In one embodiment, the processor 1210 may determine a time of a maximum point of a propagation wave component and a time of a maximum point of a reflection wave component by analyzing a secondary differential signal 1400 of the pulse wave signal 1300; and may determine a pulse wave signal amplitude corresponding to each time of the maximum points of the propagation wave component and the reflection wave component. Further, the processor 1210 may extract the first feature value and/or the second feature value by combining the time of the maximum point of the propagation wave component, the pulse wave signal amplitude corresponding to the time of the maximum point of the propagation wave component, the time of the maximum point of the reflection wave component, the pulse wave signal amplitude corresponding to the time of the maximum point of the reflection wave component, and the like.

Hereinafter, an example of feature values (e.g., the first feature value and the second feature value) extracted in the first operation mode will be described with reference to FIGS. 13 to 15.

Referring to FIG. 13, a pulse wave signal 1300 may be formed by a superposition of three component pulses 1310 to 1330. Here, a reference numeral 1300 denotes a pulse wave signal; a reference numeral 1310 denotes a first component pulse (propagation wave component); a reference numeral 1320 denotes a second component pulse (a first reflection wave component); and a reference numeral 1330 denotes a third component pulse (a second reflection wave component). Further, $T_1$ denotes a time of a maximum point of the first component pulse 1310; $P_1$ denotes an amplitude of the pulse wave signal 1300 at $T_1$; $T_2$ denotes a time of a maximum point of the second component pulse 1320; $P_2$ denotes an amplitude of the pulse wave signal 1300 at $T_2$; $T_3$ denotes a time of a maximum point of the third component pulse 1330; $P_3$ denotes an amplitude of the pulse wave signal 1300 at $T_3$; $T_{max}$ denotes a time of a maximum point in a predetermined interval (e.g., a first interval) of the pulse wave signal 1300; $P_{max}$ denotes an amplitude of the pulse wave signal 1300 at $T_{max}$; $T_{sys}$ denotes a time of a middle point between $T_1$ and $T_{max}$; $P_{sys}$ denotes an amplitude of the pulse wave signal 1300 at $T_{sys}$; $\tau_{dur}$ denotes a system setting factor $0 \leq \tau_{dur} \leq 1$ (e.g., 0.7); and $P_{area}$ denotes a sum of amplitudes of the pulse wave signal 1300 in an interval from 0 to $\tau_{dur} * T_{period}$ (e.g., a second interval).

The first feature is a feature associated with cardiac output, and may include, for example, values obtained by $P_{max}/P_{area}$, $P_{max}/P_3$, $P_{sys}/P_3$, $P_1/P_3$, $P_2/P_3$, $P_2/P_1$, and the like.

The second feature is a feature associated with total peripheral resistance, and may include, for example, values obtained by $1/(T_3-T_{sys})$, $1/(T_3-T_{max})$, $1/(T_3-T_1)$, $1/(T_3-T_2)$, $P_3/P_1$, $P_2/P_1$, and the like.

While $T_{sys}$ denotes the time of the middle point between $T_1$ and $T_{max}$ in FIG. 3, $T_{sys}$ is not limited thereto. That is, $T_{sys}$ may be an internally dividing point between $T_1$ and $T_{max}$, and an internally dividing point between $T_1$ and $T_2$.

Referring to FIG. 14, $P_n$ ($P_1$, $P_2$, $P_3$) and $T_n$ ($T_1$, $T_2$, $T_3$) of FIG. 13 may be obtained based on the secondary differential signal 1400 of the pulse wave signal 1300. Once the secondary differential signal 1400 is generated by performing secondary differentiation on the pulse wave signal 1300, the secondary differential signal 1400 may include a plurality of local minimum points min1 to min3. By arranging the local minimum points min1 to min3, included in the secondary differential signal 1400, in time-sequential order, the first local minimum point min1 corresponds to $T_1$, the second local minimum point min2 corresponds to $T_2$, and the third local minimum point min3 corresponds to $T_3$. Further, the amplitude of the pulse wave signal 1300 at $T_1$ corresponds to Pi; the amplitude of the pulse wave signal 1300 at $T_2$ corresponds to $P_2$; and the amplitude of the pulse wave signal 1300 at $T_3$ corresponds to $P_3$.

Referring to FIG. 15, $P_{max}$ and $T_{max}$ of FIG. 13 may be obtained based on the secondary differential signal 1400 of the pulse wave signal 1300. Once the secondary differential signal 1400 is generated by performing secondary differentiation on the pulse wave signal 1300, the secondary differential signal 1400 may include a plurality of local maximum points max1 to max3. By arranging the local maximum points max1 to max3, included in the secondary differential signal 1400, in time-sequential order, and by defining a time of the third local maximum point max3, among the first to the third local maximum points max1 to max3, as $T_{range}$, a search area for $P_{max}$ (i.e., a first interval) may be determined to be $0 \leq$ time $\leq T_{range}$. The processor 1210 may search for $P_{max}$ in the search area. In this case, within the search area for $P_{max}$ (first interval) ($0 \leq$ time $\leq T_{range}$), a time of a maximum point of the pulse wave signal 1300 corresponds to $T_{max}$, and an amplitude of the pulse wave signal 1300 at $T_{max}$ corresponds to $P_{max}$.

The processor 1210 may estimate cardiovascular information by using the extracted first feature value and/or second feature value as the cardiovascular feature value. In one embodiment, the processor 1210 may use a cardiovascular information estimation model which defines a relationship between the cardiovascular feature value and cardiovascular information.

<Second operation mode>

The processor 1210 may determine a reference point on the pulse wave signal 1300 in the second operation mode. In one embodiment, the processor 1210 may detect a pulse wave start point or a maximum ascending slope point from the pulse wave signal 1300; and may determine, as the reference point, one of the following: a point after a first duration from the pulse wave start point, the maximum ascending slope point, and a point before/after a second duration from the maximum ascending slope point. For example, as described above with reference to FIG. 5, the processor 1210 may detect pulse wave start points 410 and 430 or a maximum ascending slope point 420. In this case, the pulse wave start point 410 may indicate a minimum point of the pulse wave signal 1300, and the pulse wave start point 430 may indicate an intersection point 430 between a tangent line at the maximum ascending slope point 420 of the pulse wave signal 1300 and a height of the minimum point 410 of the pulse wave signal 1300. That is, the processor 1210 may detect, as the pulse wave start point, the minimum point 410 of the pulse wave signal, or the intersection point 430 between the tangent line at the maximum ascending slope point 420 of the pulse wave signal and the height of the minimum point 410 of the pulse wave signal. Further, the processor 1210 may determine, as the reference point, one of the following: a point 440 after a predetermined duration Ldur1 from the pulse wave start point 410, a point 450 after a predetermined duration Ldur2 from the pulse wave start point 430, the maximum ascending slope point 420, and points 460 and 470 before/after a predetermined duration Ldur3 from the maximum ascending slope point 420.

The processor 1210 may normalize the pulse wave signal. In one embodiment, the processor 1210 may normalize the pulse wave signal based on at least one of the following: a pulse wave signal amplitude at a point after a third duration from the pulse wave start point; a pulse wave signal amplitude at the maximum ascending slope point; a pulse wave signal amplitude at a point before/after a fourth duration from the maximum ascending slope point; and a duration of the pulse wave signal at a point higher than the reference point.

The processor 1210 may determine an area of the pulse wave signal which is at the point higher than the reference point on the normalized pulse wave signal, and may estimate cardiovascular information by using the determined area as a cardiovascular feature value. In one embodiment, the processor 1210 may use a cardiovascular information model which defines a relationship between the cardiovascular feature value and cardiovascular information.

In one embodiment, in order to consider only reflection wave component information, the processor 1210 may remove an area of the propagation wave component, included in the area of the pulse wave signal at the point higher than the reference point, from the area of the pulse wave signal, and may estimate cardiovascular information by using the area, from which an effect of the propagation wave component is removed, as a cardiovascular feature value. For example, the processor 1210 may determine an area of the propagation wave component based on the determined point of the pulse wave signal and the reference point; and may remove the area of the propagation wave component from the area of the pulse wave signal which is higher than the reference point. In this case, the predetermined point may be a point at which the propagation wave component included in the pulse wave signal may appear, and may be obtained experimentally as a preset value.

In addition, upon normalizing the pulse wave signal as described above, the processor 1210 may also calculate the area of the pulse wave signal which is at the point higher than the reference point on the normalized pulse wave signal; and upon calculating the area of the pulse wave signal which is at the point higher than the reference point, the processor 1210 may also normalize the calculated area.

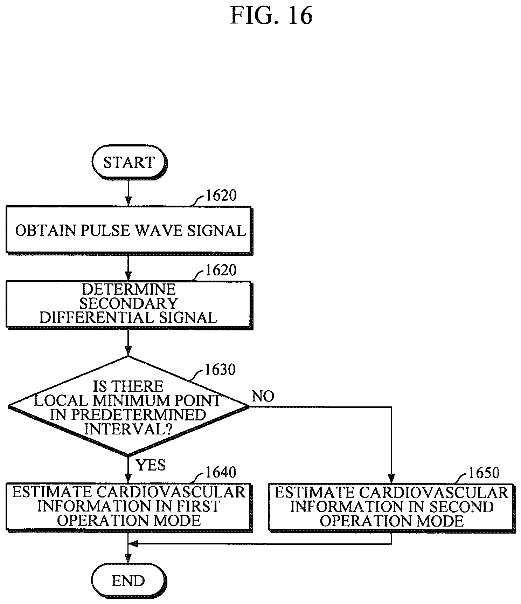

FIG. 16 is a diagram illustrating a method of estimating cardiovascular information according to another example embodiment. The cardiovascular information estimating method of FIG. 16 may be performed by the cardiovascular information estimating apparatus 1200 of FIG. 12.

Referring to FIG. 16, the cardiovascular information estimating apparatus 1200 may obtain a pulse wave signal of an object in operation 1610. Here, the pulse wave signal may be a PPG signal. For example, the cardiovascular information estimating apparatus 1200 may obtain the pulse wave signal of the object by receiving the pulse wave signal from an external device which measures and/or stores pulse wave signals, or may obtain the pulse wave signal by emitting light of a predetermined wavelength and receiving light reflected or scattered from the object.

The cardiovascular information estimating apparatus 1200 may determine a secondary differential signal of the pulse wave signal in operation 1620, and may determine whether there is a local minimum point in a predetermined interval of the secondary differential signal in operation 1630. In this case, the predetermined interval may be an interval in which the propagation wave component included in the pulse wave signal may appear, and may be obtained experimentally as a preset value.

If there is a local minimum point in the predetermined interval of the secondary differential signal, the cardiovascular information estimating apparatus 1200 may estimate cardiovascular information of the object in the first operation mode in operation 1640.

If there is no local minimum point in the predetermined interval of the secondary differential signal, the cardiovascular information estimating apparatus 1200 may estimate cardiovascular information of the object in the second operation mode in operation 1650.

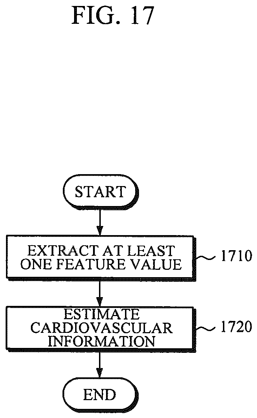

FIG. 17 is a diagram illustrating a method of estimating cardiovascular information in a first operation mode according to an example embodiment. The method of FIG. 17 may be an example of the estimating of cardiovascular information in the first operation mode in operation 1640 of FIG. 16.

Referring to FIG. 17, the cardiovascular information estimating apparatus 1200 may extract at least one feature value from a pulse wave signal in operation 1710. In this case, the feature value may include a first feature value and a second feature value, in which the first feature value may be associated with cardiac output, and the second feature value may be associated with total peripheral resistance. In one embodiment, the cardiovascular information estimating apparatus 1200 may analyze a secondary differential signal of the pulse wave signal to determine a time of a maximum point of a propagation wave component and a time of a maximum point of a reflection wave component; and may determine a pulse wave signal amplitude corresponding to each time of the maximum points of the propagation wave component and the reflection wave component. Further, the cardiovascular information estimating apparatus 1200 may extract at least one feature value by combining the time of the maximum point of the propagation wave component, the pulse wave signal amplitude corresponding to the time of the maximum point of the propagation wave component, the time of the maximum point of the reflection wave component, the pulse wave signal amplitude corresponding to the time of the maximum point of the reflection wave component, and the like. The first feature value and the second feature value are described above in detail with reference to FIGS. 13 to 15, such that detailed description thereof will be omitted.

The cardiovascular information estimating apparatus 1200 may estimate cardiovascular information by using the extracted at least one feature value as a cardiovascular feature value in operation 1720. In one embodiment, the cardiovascular information estimating apparatus 1200 may use a cardiovascular information estimation model which defines a relationship between the cardiovascular feature value and cardiovascular information.

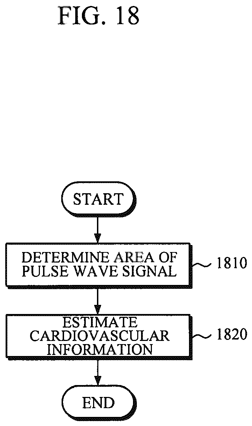

FIG. 18 is a diagram illustrating a method of estimating cardiovascular information in a second operation mode according to an example embodiment. The method of FIG. 18 may be an example of the estimating of cardiovascular information in the second operation mode in operation 1650 of FIG. 16.

Referring to FIG. 18, the cardiovascular information estimating apparatus 1200 may determine an area of a pulse wave signal which is at a point higher than a reference point in operation 1810. In this case, as described above, the reference point may be a point of reference for determining an area to be used as a cardiovascular feature value.

In one embodiment, the cardiovascular information estimating apparatus 1200 may determine the reference point on the pulse wave signal, may normalize the pulse wave signal, and then may determine the area of the pulse wave signal which is at the point higher than the reference point on the normalized pulse wave signal.

More specifically, the cardiovascular information estimating apparatus 1200 may detect a pulse wave pulse wave start point or a maximum ascending slope point from the pulse wave signal; and may determine, as the reference point, one of the following: a point after a first duration from the pulse wave start point, the maximum ascending slope point, and a point before/after a second duration from the maximum ascending slope point. In this case, the pulse wave start point may indicate a minimum point of the pulse wave signal, or an intersection point between a tangent line at the maximum ascending slope point of the pulse wave signal and a height of the minimum point of the pulse wave signal. The cardiovascular information estimating apparatus 1200 may normalize the pulse wave signal based on at least one of the following: a pulse wave signal amplitude at a point after a third duration from the pulse wave start point; a pulse wave signal amplitude at the maximum ascending slope point; a pulse wave signal amplitude at a point before/after a fourth duration from the maximum ascending slope point; and a duration of the pulse wave signal at the point higher than the reference point. The cardiovascular information estimating apparatus 1200 may determine an area of the pulse wave signal which is at the point higher than the reference point on the normalized pulse wave signal.

The cardiovascular information estimating apparatus 1200 may estimate cardiovascular information by using the determined area of the pulse wave signal, which is at the point higher than the reference point, as a cardiovascular feature value in operation 1820. In one embodiment, the cardiovascular information estimating apparatus 1200 may use a cardiovascular information model which defines a relationship between the cardiovascular feature value and cardiovascular information.

Further, in one embodiment, in order to consider only reflection wave component information, the cardiovascular information estimating apparatus 1200 may remove an area of the propagation wave component, included in the area of the pulse wave signal at the point higher than the reference point, from the area of the pulse wave signal, and may estimate cardiovascular information by using the area, from which an effect of the propagation wave component is removed, as a cardiovascular feature value. For example, the cardiovascular information estimating apparatus 1200 may determine an area of the propagation wave component based on the determined point of the pulse wave signal and the reference point; and may remove the area of the propagation wave component from the area of the pulse wave signal which is at the point higher than the reference point. In this case, the predetermined point may be a point at which the propagation wave component included in the pulse wave signal may appear, and may be obtained experimentally as a preset value.

In addition, as described above, upon normalizing the pulse wave signal, the cardiovascular information estimating apparatus 1200 may also calculate the area of the pulse wave signal which is at the point higher than the reference point on the normalized pulse wave signal; and upon calculating the area of the pulse wave signal which is at the point higher than the reference point, the cardiovascular information estimating apparatus 1200 may also normalize the calculated area.

While not restricted thereto, an example embodiment can be embodied as computer-readable code on a computer-readable recording medium. The computer-readable recording medium is any data storage device that can store data that can be thereafter read by a computer system. Examples of the computer-readable recording medium include read-only memory (ROM), random-access memory (RAM), CD-ROMs, magnetic tapes, floppy disks, and optical data storage devices. The computer-readable recording medium can also be distributed over network-coupled computer systems so that the computer-readable code is stored and executed in a distributed fashion. Also, an example embodiment may be written as a computer program transmitted over a computer-readable transmission medium, such as a carrier wave, and received and implemented in general-use or special-purpose digital computers that execute the programs. Moreover, it is understood that in example embodiments, one or more units of the above-described apparatuses and devices can include circuitry, a processor, a microprocessor, etc., and may execute a computer program stored in a computer-readable medium.

The foregoing exemplary embodiments are merely exemplary and are not to be construed as limiting. The present teaching can be readily applied to other types of apparatuses. Also, the description of the exemplary embodiments is intended to be illustrative, and not to limit the scope of the claims, and many alternatives, modifications, and variations will be apparent to those skilled in the art.

**Claims**

1. An apparatus (200) for estimating cardiovascular information, the apparatus comprising:

a memory (720) storing instructions; and
a processor (220) configured to execute the instructions to:

obtain (810) a pulse wave signal (100; 410) of an object, wherein the pulse wave signal being formed by a superposition of a propagation wave component and a plurality of reflection wave components; determine (820) an area under the pulse wave signal and above a reference point (610) on the pulse wave signal;
determine (1620) a secondary differential signal (420) of the pulse wave signal;
analyze the secondary differential signal to:

determine a local minimum point in a predetermined interval of the secondary differential signal, and
determine a time of the local minimum point to be the time of a maximum point of the propagation wave component;

determine (1630) a point of the pulse wave signal, which corresponds to the time of the local minimum point;
determine an area of the propagation wave component included in the determined area of the pulse wave signal based on the determined point of the pulse wave signal and the reference point;
remove the determined area of the propagation wave component from the determined area of the pulse wave signal; and estimate (830) cardiovascular information of the object based on the determined area of the pulse wave signal from which the determined area of the the propagation wave component is removed.

2. The apparatus of claim 1, further comprising a pulse wave signal obtainer comprising:

a light source (310) configured to emit light onto the object; and
a photodetector (320) configured to obtain the pulse wave signal by receiving the light returning from the object,
wherein the pulse wave signal obtainer (210) is configured to provide the pulse wave signal to the processor.

3. The apparatus of claim 1 or 2, wherein the determined point of the pulse wave signal is the reference point.

4. The apparatus of one of claims 1 to 3, wherein the processor is further configured to execute the instructions to detect a pulse wave start point or a maximum ascending slope point (520) from the pulse wave signal, and determine, as the reference point, one of a point after a first duration from the pulse wave start point, the maximum ascending slope point, and a point before or after a second duration from the maximum ascending slope point.

5. The apparatus of claim 4, wherein the processor is further configured to execute the instructions to detect, as the pulse wave start point, a minimum point (510) of the pulse wave signal, or an intersection point (530) between a tangent line at the maximum ascending slope point of the pulse wave signal and a height of the minimum point of the pulse wave signal.

6. The apparatus of one of claims 1 to 5, wherein the processor is further configured to execute the instructions to normalize (920) the pulse wave signal or the determined area of the pulse wave signal based on at least one of a pulse wave signal amplitude corresponding to a time of a maximum point of a propagation wave component included in the pulse wave signal, a pulse wave signal amplitude at a point after a third duration from a pulse wave start point, a pulse wave signal amplitude at a maximum ascending slope point, a pulse wave signal amplitude at a point before or after a fourth duration from the maximum ascending slope point, and a duration of the pulse wave signal at a point higher than the reference point, or
wherein the cardiovascular information comprises at least one of blood pressure, vascular age, arterial stiffness, vascular compliance, blood glucose, blood triglyceride, and total peripheral resistance.

7. The apparatus of one of claims 1 to 6, wherein the processor is further configured to execute the instructions to estimate the cardiovascular information by using the determined area of the pulse wave signal from which the area of the the propagation wave component is removed as a cardiovascular feature value.

8. The apparatus of claim 7, wherein the processor is further configured to execute the instructions to estimate the cardiovascular information by using a cardiovascular information estimation model which defines a relationship between the cardiovascular feature value and the cardiovascular information.

9. The apparatus of one of claims 1 to 8, wherein the processor is further configured to execute the instructions to remove (815) noise from the obtained pulse wave signal.

10. A method of estimating cardiovascular information, the method comprising:

obtaining (1620) a pulse wave signal of an object, wherein the pulse wave signal being formed by a superposition of a propagation wave component and a plurality of reflection wave components;
determining (820) an area under the pulse wave signal and above a reference point (610) on the pulse wave signal;
determining (1620) a secondary differential signal of the pulse wave signal;
analyzing the secondary differential signal to:

determining a local minimum point in a predetermined interval of the secondary differential signal, and
determining a time of the local minimum point to be the time of a maximum point of the propagation wave component;

determining (1630) a point of the pulse wave signal, which corresponds to the time of the local minimum point;
determining an area of the propagation wave component included in the determined area of the pulse wave signal based on the determined point of the pulse wave signal and the reference point;
removing the determined area of the propagation wave component from the determined area of the pulse wave signal; and
estimating (830) cardiovascular information of the object based on the determined area of the pulse wave signal from which the determined area of the the propagation wave component is removed.

11. The method of claim 10, wherein the obtaining the pulse wave signal of the object comprises emitting light onto the object; and obtaining the pulse wave signal by receiving the light returning from the object, or
wherein the estimating the cardiovascular information in comprises extracting at least one feature value by analyzing the secondary differential signal; and estimating the cardiovascular information by using the extracted at least one feature value as a cardiovascular feature value.

12. The method of claim 11, wherein the extracting the at least one feature value comprises:

determining a maximum point of a reflection wave component by analyzing the secondary differential signal;
determining a pulse wave signal amplitude corresponding to a time of the maximum point of the reflection wave component; and
extracting the at least one feature value by combining at least one of the time of the maximum point of the propagation wave component, a pulse wave signal amplitude corresponding to the time of the maximum point of the propagation wave component, the time of the maximum point of the reflection wave component, and a pulse wave signal amplitude corresponding to the time of the maximum point of the reflection wave component.

13. The method of claim 10, wherein the determining the area of the pulse wave signal comprises:

detecting a pulse wave start point or a maximum ascending slope point from the pulse wave signal; and
determining, as the reference point, one of a point after a first duration from the pulse wave start point, the maximum ascending slope point, and a point before or after a second duration from the maximum ascending slope point.

14. The method of claim 13, wherein the detecting the pulse wave start point or the maximum ascending slope point comprises detecting, as the pulse wave start point, a minimum point (510) of the pulse wave signal, or an intersection point (530) between a tangent line at the maximum ascending slope point of the pulse wave signal and a height of the minimum point of the pulse wave signal.

15. The method of claim 10, wherein the estimating the cardiovascular information comprises normalizing the pulse wave signal or the determined area of the pulse wave signal based on at least one of a pulse wave signal amplitude at the point after a first duration from a pulse wave start point, a pulse wave signal amplitude at a maximum ascending slope point, a pulse wave signal amplitude at a point before or after a second duration from the maximum ascending slope point, and a duration of the pulse wave signal at a point higher than the reference point.

**Patentansprüche**

1. Eine Vorrichtung (200) zum Schätzen von kardiovaskulären Informationen, wobei die Vorrichtung umfasst:

einen Speicher (720), der Anweisungen speichert; und
einen Prozessor (220), der so konfiguriert ist, dass er die Anweisungen ausführt, zum:

Erhalten (810) eines Impulswellensignals (100; 410) eines Objekts, wobei das Impulswellensignal durch eine Überlagerung einer Ausbreitungswellenkomponente und einer Vielzahl von Reflexionswellenkomponenten gebildet wird;
Bestimmen (820) einer Fläche unter dem Impulswellensignal und über einem Referenzpunkt (610) auf dem Impulswellensignal;
Bestimmen (1620) eines sekundären Differenzsignals (420) des Impulswellensignals;
Analysieren des sekundären Differenzsignals:

Bestimmen eines lokalen Minimalpunktes in einem vorbestimmten Intervall des sekundären Differenzsignals, und
Bestimmen eines Zeitpunkts des lokalen Minimalpunkts als Zeitpunkt eines Maximalpunkts der Ausbreitungswellenkomponente;

Bestimmen (1630) eines Punktes des Impulswellensignals, der dem Zeitpunkt des lokalen Minimalpunktes entspricht;
Bestimmen einer Fläche der Ausbreitungswellenkomponente, die in der bestimmten Fläche des Impulswellensignals enthalten ist, basierend auf dem bestimmten Punkt des Impulswellensignals und dem Referenzpunkt;
Entfernen des ermittelten Bereichs der Ausbreitungswellenkomponente aus dem ermittelten Bereich des Impulswellensignals; und
Schätzen (830) kardiovaskulärer Informationen des Objekts auf der Grundlage des ermittelten Bereichs des Impulswellensignals, aus dem der ermittelte Bereich der Ausbreitungswellenkomponente entfernt wurde.

2. Vorrichtung nach Anspruch 1, die ferner einen Pulswellensignalgeber umfasst, der Folgendes umfasst:

eine Lichtquelle (310), die so konfiguriert ist, dass sie Licht auf das Objekt emittiert; und
einen Fotodetektor (320), der so konfiguriert ist, dass er das Impulswellensignal erhält, indem er das von dem Objekt zurückkehrende Licht empfängt,
wobei der Pulswellensignalempfänger (210) so konfiguriert ist, dass er das Impulswellensignal an den Prozessor liefert.

3. Vorrichtung nach Anspruch 1 oder 2, wobei der ermittelte Punkt des Impulswellensignals ist der Referenzpunkt.

4. Vorrichtung nach einem der Ansprüche 1 bis 3, wobei der Prozessor ferner so konfiguriert ist, dass er die Anweisungen ausführt, um einen Pulswellen-Startpunkt oder einen Punkt maximaler Steigung (520) aus dem Impulswellensignal zu erkennen und als Bezugspunkt einen Punkt nach einer ersten Dauer ab dem Pulswellen-Startpunkt, den Punkt maximaler Steigung oder einen Punkt vor oder nach einer zweiten Dauer ab dem Punkt maximaler Steigung zu bestimmen.

5. Vorrichtung nach Anspruch 4, wobei der Prozessor ferner so konfiguriert ist, dass er die Befehle ausführt, um als Pulswellen-Startpunkt einen Minimalpunkt (510) des Impulswellensignals oder einen Schnittpunkt (530) zwischen einer Tangente am Punkt der maximalen ansteigenden Steigung des Impulswellensignals und einer Höhe des Minimalpunkts des Impulswellensignals zu erfassen.

6. Vorrichtung nach einem der Ansprüche 1 bis 5, wobei der Prozessor ferner so konfiguriert ist, dass er die Befehle ausführt, um das Impulswellensignal oder den bestimmten Bereich des Impulswellensignals auf der Grundlage von mindestens einer Impulswellensignalamplitude, die einem Zeitpunkt eines Maximalpunkts einer in dem Impulswellensignal enthaltenen Ausbreitungswellenkomponente entspricht, zu normalisieren (920) einer Impulswellensignal-Amplitude an einem Punkt nach einer dritten Dauer ab einem Impulswellen-Startpunkt, einer Impulswellensignal-Amplitude an einem Punkt mit maximaler Steigung, einer Impulswellensignal-Amplitude an einem Punkt vor oder nach einer vierten Dauer ab dem Punkt mit maximaler Steigung und einer Dauer des Impulswellensignals an einem Punkt, der höher als der Referenzpunkt ist, oder
wobei die kardiovaskulären Informationen mindestens eines der folgenden Elemente umfassen: Blutdruck, Gefäßalter, Arteriensteifigkeit, Gefäßcompliance, Blutzucker, Bluttriglyceride und gesamter peripherer Widerstand.

7. Vorrichtung nach einem der Ansprüche 1 bis 6, wobei der Prozessor ferner so konfiguriert ist, dass er die Anweisungen ausführt, um die kardiovaskulären Informationen zu schätzen, indem er den bestimmten Bereich des Impulswellensignals, aus dem der Bereich der Ausbreitungswellenkomponente entfernt wird, als kardiovaskulären Merkmalswert verwendet.

8. Vorrichtung nach Anspruch 7, wobei der Prozessor ferner so konfiguriert ist, dass er die Anweisungen ausführt, um die kardiovaskulären Informationen unter Verwendung eines Schätzmodells für kardiovaskuläre Informationen zu schätzen, das eine Beziehung zwischen dem kardiovaskulären Merkmalswert und den kardiovaskulären Informationen definiert.

9. Vorrichtung nach einem der Ansprüche 1 bis 8, wobei der Prozessor ferner so konfiguriert ist, dass er die Anweisungen zum Entfernen (815) von Rauschen aus dem erhaltenen Impulswellensignal ausführt.

10. Ein Verfahren zur Schätzung von kardiovaskulären Informationen, wobei das Verfahren umfasst:

Erhalten (1620) eines Impulswellensignals eines Objekts, wobei das Impulswellensignal durch eine Überlagerung einer Ausbreitungswellenkomponente und einer Vielzahl von Reflexionswellenkomponenten gebildet wird;
Bestimmung (820) einer Fläche unter dem Impulswellensignal und über einem Referenzpunkt (610) auf dem Impulswellensignal;
Bestimmung (1620) eines sekundären Differenzsignals des Impulswellensignals;
Analysieren des sekundären Differenzsignals:

Bestimmen eines lokalen Minimalpunktes in einem vorbestimmten Intervall des sekundären Differenzsignals, und
Bestimmung eines Zeitpunkts des lokalen Minimalpunkts als Zeitpunkt eines Maximalpunkts der Ausbreitungswellenkomponente;

Bestimmung (1630) eines Punktes des Impulswellensignals, der dem Zeitpunkt des lokalen Minimalpunktes entspricht;
Bestimmen einer Fläche der Ausbreitungswellenkomponente, die in der bestimmten Fläche des Impulswellensignals enthalten ist, basierend auf dem bestimmten Punkt des Impulswellensignals und dem Referenzpunkt;
Entfernen des ermittelten Bereichs der Ausbreitungswellenkomponente aus dem ermittelten Bereich des Impulswellensignals; und
Schätzen (830) von kardiovaskulären Informationen des Objekts auf der Grundlage des bestimmten Bereichs des Impulswellensignals, aus dem der bestimmte Bereich der Ausbreitungswellenkomponente entfernt wird.

11. Verfahren nach Anspruch 10, wobei das Erhalten des Impulswellensignals des Objekts das Emittieren von Licht auf das Objekt und das Erhalten des Impulswellensignals durch Empfangen des von dem Objekt zurückkehrenden Lichts umfasst, oder wobei das Schätzen der kardiovaskulären Information das Extrahieren von mindestens einem Merkmalswert durch Analysieren des sekundären Differenzsignals und das Schätzen der kardiovaskulären Information durch Verwenden des extrahierten mindestens einen Merkmalswertes als kardiovaskulären Merkmalswert umfasst.

12. Verfahren nach Anspruch 11, wobei das Extrahieren des mindestens einen Merkmalswertes umfasst:

Bestimmung eines Maximalpunktes einer Reflexionswellenkomponente durch Analyse des sekundären Differenzsignals;
Bestimmung einer Impulswellensignalamplitude, die einem Zeitpunkt des Maximalpunkts der Reflexionswellenkomponente entspricht; und
Extrahieren des mindestens einen Merkmalswerts durch Kombinieren mindestens eines der folgenden Elemente: Zeitpunkt des Maximalpunkts der Ausbreitungswellenkomponente, eine Impulswellensignalamplitude, die dem Zeitpunkt des Maximalpunkts der Ausbreitungswellenkomponente entspricht, der Zeitpunkt des Maximalpunkts der Reflexionswellenkomponente und eine Impulswellensignalamplitude, die dem Zeitpunkt des Maximalpunkts der Reflexionswellenkomponente entspricht.

13. Verfahren nach Anspruch 10, wobei die Bestimmung der Fläche des Impulswellensignals umfasst:

Erfassen eines Pulswellen-Startpunkts oder eines maximalen Anstiegspunkts aus dem Impulswellensignal; und
Bestimmen eines Punktes nach einer ersten Dauer ab dem Startpunkt der Pulswelle, des Punktes der maximalen ansteigenden Steigung und eines Punktes vor oder nach einer zweiten Dauer ab dem Punkt der maximalen ansteigenden Steigung als Referenzpunkt.

14. Verfahren nach Anspruch 13, wobei das Erfassen des Pulswellen-Startpunktes oder des Punktes der maximalen ansteigenden Steigung das Erfassen eines Minimalpunktes (510) des Impulswellensignals oder eines Schnittpunktes (530) zwischen einer Tangente am Punkt der maximalen ansteigenden Steigung des Impulswellensignals und einer Höhe des Minimalpunktes des Impulswellensignals als den Pulswellen-Startpunkt umfasst.

15. Verfahren nach Anspruch 10, wobei das Schätzen der kardiovaskulären Informationen das Normalisieren des Impulswellensignals oder des bestimmten Bereichs des Impulswellensignals auf der Grundlage mindestens einer der folgenden Informationen umfasst: eine Pulswellensignalamplitude an dem Punkt nach einer ersten Dauer ab einem Pulswellenstartpunkt, eine Pulswellensignalamplitude an einem Punkt mit maximaler Steigung, eine Pulswellensignalamplitude an einem Punkt vor oder nach einer zweiten Dauer ab dem Punkt mit maximaler Steigung und eine Dauer des Impulswellensignals an einem Punkt, der höher als der Referenzpunkt ist.

**Revendications**

1. Appareil (200) d'estimation d'informations cardiovasculaires, l'appareil comprenant :

une mémoire (720) stockant des instructions ; et
un processeur (220) configuré pour exécuter les instructions afin :

d'obtenir (810) un signal d'onde pulsée (100; 410) d'un sujet, le signal d'onde pulsée étant formé par la superposition d'une composante d'onde de propagation et d'une pluralité de composantes d'onde de réflexion ;
de déterminer (820) une zone située sous le signal d'onde pulsée et au-dessus d'un point de référence (610) sur le signal d'onde pulsée ;
de déterminer (1620) un signal différentiel secondaire (420) du signal d'onde d'impulsion ;
d'analyser le signal différentiel secondaire pour :

déterminer un point minimum local dans un intervalle prédéterminé du signal différentiel secondaire, et

déterminer l'heure du point minimum local comme étant l'heure du point maximum de la composante de l'onde de propagation ;

de déterminer (1630) un point du signal d'onde d'impulsion qui correspond à l'instant du point minimum local ;

de déterminer une zone de la composante d'onde de propagation incluse dans la zone déterminée du signal d'onde d'impulsion sur la base du point déterminé du signal d'onde d'impulsion et du point de référence ;

de retirer la zone déterminée de la composante d'onde de propagation de la zone déterminée du signal d'onde d'impulsion ; et

d'estimer (830) les informations cardiovasculaires du sujet sur la base de la zone déterminée du signal d'onde d'impulsion dont la zone déterminée de la composante d'onde de propagation est retirée.

2. Appareil selon la revendication 1, comprenant en outre un dispositif d'obtention d'un signal d'onde d'impulsion comprenant :

une source de lumière (310) configurée pour émettre de la lumière sur le sujet; et

un photodétecteur (320) configuré pour obtenir le signal d'onde d'impulsion en recevant la lumière renvoyée par le sujet,

dans lequel le dispositif d'obtention du signal d'onde d'impulsion (210) est configuré pour fournir le signal d'onde d'impulsion au processeur.

3. Appareil selon les revendications 1 ou 2, dans lequel le point déterminé du signal d'onde d'impulsion est le point de référence.

4. Appareil selon l'une des revendications 1 à 3, dans lequel le processeur est en outre configuré pour exécuter les instructions afin de détecter un point de départ de l'onde d'impulsion ou un point de pente ascendante maximale (520) à partir du signal d'onde d'impulsion, et de déterminer, en tant que point de référence, l'un des points suivants : un point après une première durée à partir du point de départ de l'onde d'impulsion, le point de pente ascendante maximale, et un point avant ou après une deuxième durée à partir du point de pente ascendante maximale.

5. Appareil selon la revendication 4, dans lequel le processeur est en outre configuré pour exécuter les instructions afin de détecter, en tant que point de départ de l'onde d'impulsion, un point minimum (510) du signal d'onde d'impulsion, ou un point d'intersection (530) entre une ligne tangente au point de pente ascendante maximale du signal d'onde d'impulsion et une hauteur du point minimum du signal d'onde d'impulsion.

6. Appareil selon l'une des revendications 1 à 5, dans lequel le processeur est en outre configuré pour exécuter les instructions afin de normaliser (920) le signal d'onde d'impulsion ou la zone déterminée du signal d'onde d'impulsion sur la base d'au moins l'un des éléments suivants une amplitude de signal d'onde d'impulsion correspondant à un moment d'un point maximum d'une composante d'onde de propagation incluse dans le signal d'onde d'impulsion, une amplitude du signal d'onde d'impulsion à un point situé après une troisième durée à partir d'un point de départ de l'onde d'impulsion, une amplitude du signal d'onde d'impulsion à un point de pente ascendante maximale, une amplitude du signal d'onde d'impulsion à un point situé avant ou après une quatrième durée à partir du point de pente ascendante maximale, et une durée du signal d'onde d'impulsion à un point plus élevé que le point de référence, ou dans lequel les informations cardiovasculaires comprennent au moins l'un des éléments suivants : pression artérielle, âge vasculaire, rigidité artérielle, compliance vasculaire, glucose sanguin, triglycérides sanguins et résistance périphérique totale.

7. Appareil selon l'une des revendications 1 à 6, dans lequel le processeur est en outre configuré pour exécuter les instructions afin d'estimer les informations cardiovasculaires en utilisant la zone déterminée du signal d'onde d'impulsion à partir de laquelle la zone de la composante d'onde de propagation est supprimée comme valeur de caractéristique cardiovasculaire.

8. Appareil selon la revendication 7, dans lequel le processeur est en outre configuré pour exécuter les instructions afin d'estimer les informations cardiovasculaires à l'aide d'un modèle d'estimation des informations cardiovasculaires qui définit une relation entre la valeur de la caractéristique cardiovasculaire et les informations cardiovasculaires.

9. Appareil selon l'une des revendications 1 à 8, dans lequel le processeur est en outre configuré pour exécuter les instructions afin d'éliminer (815) le bruit du signal d'onde d'impulsion obtenu.

10. Procédé d'estimation d'informations cardiovasculaires, comprenant les opérations suivantes :

obtenir (1620) un signal d'onde pulsée d'un sujet, le signal d'onde pulsée étant formé par la superposition d'une composante d'onde de propagation et d'une pluralité de composantes d'onde de réflexion ;

déterminer (820) une zone située sous le signal d'onde de pouls et au-dessus d'un point de référence (610) sur le signal d'onde de pouls ;

déterminer (1620) un signal différentiel secondaire du signal d'onde d'impulsion;

analyser le signal différentiel secondaire pour :

déterminer un point minimum local dans un intervalle prédéterminé du signal différentiel secondaire, et

déterminer l'heure du point minimum local comme étant l'heure du point maximum de la composante de l'onde de propagation ;

déterminer (1630) un point du signal d'onde d'impulsion qui correspond à l'instant du point minimum local ;

déterminer une zone de la composante d'onde de propagation incluse dans la zone déterminée du signal d'onde d'impulsion sur la base du point déterminé du signal d'onde d'impulsion et du point de référence ;

retirer la zone déterminée de la composante d'onde de propagation de la zone déterminée du signal d'onde d'impulsion ; et

estimer (830) des informations cardiovasculaires du sujet sur la base de la zone déterminée du signal d'onde d'impulsion dont la zone déterminée de la composante d'onde de propagation est supprimée.

**11.** Procédé selon la revendication 10, dans lequel l'obtention du signal d'onde d'impulsion du sujet comprend l'émission de lumière sur le sujet ; et l'obtention du signal d'onde d'impulsion par la réception de la lumière revenant du sujet, ou

dans lequel l'estimation des informations cardiovasculaires comprend l'extraction d'au moins une valeur caractéristique par l'analyse du signal différentiel secondaire ; et l'estimation des informations cardiovasculaires par l'utilisation d'au moins une valeur caractéristique extraite en tant que valeur caractéristique cardiovasculaire.

**12.** Procédé selon la revendication 11, dans lequel l'extraction d'au moins une valeur de caractéristique comprend les opérations suivantes :

déterminer un point maximum d'une composante de l'onde de réflexion en analysant le signal différentiel secondaire ;

déterminer l'amplitude d'un signal d'onde d'impulsion correspondant au moment du point maximum de la composante d'onde de réflexion ; et

extraire au moins une valeur caractéristique en combinant au moins l'un des éléments suivants : l'heure du point maximal de la composante onde de propagation, une amplitude de signal d'onde d'impulsion correspondant à l'heure du point maximal de la composante onde de propagation, l'heure du point maximal de la composante onde de réflexion, et une amplitude de signal d'onde d'impulsion correspondant à l'heure du point maximal de la composante onde de réflexion.

**13.** Procédé selon la revendication 10, dans lequel la détermination de la zone du signal d'onde de pouls comprend les opérations suivantes :

détecter un point de départ de l'onde d'impulsion ou d'un point de pente ascendante maximale à partir du signal d'onde d'impulsion ; et

déterminer, en tant que point de référence, l'un des points suivants : un point après une première durée à partir du point de départ de l'onde de pouls, le point de pente ascendante maximale, et un point avant ou après une deuxième durée à partir du point de pente ascendante maximale.

**14.** Procédé selon la revendication 13, dans lequel la détection du point de départ de l'onde d'impulsion ou du point de pente ascendante maximale comprend la détection, en tant que point de départ de l'onde d'impulsion, d'un point minimum (510) du signal d'onde d'impulsion, ou d'un point d'intersection (530) entre une ligne tangente au point de pente ascendante maximale du signal d'onde d'impulsion et une hauteur du point minimum du signal d'onde d'impulsion.

**15.** Procédé selon la revendication 10, dans lequel l'estimation des informations cardiovasculaires comprend la normalisation du signal d'onde de pouls ou de la zone déterminée du signal d'onde de pouls sur la base d'au moins un des éléments suivants : amplitude du signal d'onde de pouls au point après une première durée à partir d'un point de départ de l'onde de pouls, amplitude du signal d'onde de pouls à un point de pente ascendante maximale, amplitude du signal d'onde de pouls à un point avant ou après une deuxième durée à partir du point de pente ascendante maximale, et durée du signal d'onde de pouls à un point plus élevé que le point de référence.

FIG. 1

FIG. 2

FIG. 3

# FIG. 4

FIG. 5

FIG. 6

# FIG. 7

# FIG. 8

```
            ╭─────────╮
            │  START  │
            ╰────┬────╯
                 ↓
    ┌────────────────────────────┐
    │  OBTAIN PULSE WAVE SIGNAL   │──~ 810
    └────────────┬───────────────┘
                 ↓
    ┌────────────────────────────┐
    │     DETERMINE AREA OF       │──~ 820
    │     PULSE WAVE SIGNAL       │
    └────────────┬───────────────┘
                 ↓
    ┌────────────────────────────┐
    │   ESTIMATE CARDIOVASCULAR   │──~ 830
    │       INFORMATION           │
    └────────────┬───────────────┘
                 ↓
            ╭─────────╮
            │   END   │
            ╰─────────╯
```

# FIG. 9

START

DETERMINE REFERENCE POINT ~ 910

NORMALIZE PULSE WAVE SIGNAL ~ 920

DETERMINE AREA OF
PULSE WAVE SIGNAL ~ 930

END

# FIG. 10

START

DETERMINE REFERENCE POINT — 1010

DETERMINE AREA OF
PULSE WAVE SIGNAL — 1020

NORMALIZE PULSE WAVE SIGNAL — 1030

END

## FIG. 11

```
        ( START )
            |
            v
+---------------------------+
| OBTAIN PULSE WAVE SIGNAL  |~ 810
+---------------------------+
            |
            v
+---------------------------+
|       REMOVE NOISE        |~ 815
+---------------------------+
            |
            v
+---------------------------+
|    DETERMINE AREA OF      |~ 820
|    PULSE WAVE SIGNAL      |
+---------------------------+
            |
            v
+---------------------------+
| ESTIMATE CARDIOVASCULAR   |~ 830
|      INFORMATION          |
+---------------------------+
            |
            v
          ( END )
```

FIG. 12

FIG. 13

FIG. 14

FIG. 15

# FIG. 16

START

OBTAIN PULSE WAVE SIGNAL ⟋1620

DETERMINE
SECONDARY
DIFFERENTIAL SIGNAL ⟋1620

IS THERE
LOCAL MINIMUM POINT
IN PREDETERMINED
INTERVAL? ⟋1630 —NO—

YES

ESTIMATE CARDIOVASCULAR
INFORMATION IN FIRST
OPERATION MODE ⟋1640

ESTIMATE CARDIOVASCULAR
INFORMATION IN SECOND
OPERATION MODE ⟋1650

END

# FIG. 17

START

EXTRACT AT LEAST ONE FEATURE VALUE  ~ 1710

ESTIMATE CARDIOVASCULAR INFORMATION  ~ 1720

END

FIG. 18

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009326393 A1
- US 2006074322 A1
- JP 2000300526 A
- US 2009024012 A1
- WO 2018172958 A1
- JP 2000237153 A